(19) Europäisches Patentamt / European Patent Office / Office européen des brevets

(11) **EP 4 641 355 A2**

(12) **EUROPEAN PATENT APPLICATION**

(43) Date of publication:
**29.10.2025 Bulletin 2025/44**

(21) Application number: **25172314.4**

(22) Date of filing: **24.04.2025**

(51) International Patent Classification (IPC):
**G06F 3/01** (2006.01)    **G06N 3/0455** (2023.01)
**G06V 40/20** (2022.01)

(52) Cooperative Patent Classification (CPC):
**G06F 3/017**; G06N 3/0455; G06V 40/28

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **26.04.2024 US 202463639339 P**
         **10.04.2025 US 202519175941**

(71) Applicant: **Samsung Electronics Co., Ltd.**
**Suwon-si, Gyeonggi-do 16677 (KR)**

(72) Inventors:
• **ZHANG, Menghe**
  **San Jose, CA, 95134 (US)**
• **ZHAO, Dongfang**
  **San Jose, CA, 95134 (US)**
• **SOLEYMANI, Sobhan**
  **San Jose, CA, 95134 (US)**
• **LIANG, Yangwen**
  **San Jose, CA, 95134 (US)**
• **WANG, Shuangquan**
  **San Jose, CA, 95134 (US)**
• **SONG, Kee-Bong**
  **San Jose, CA, 95134 (US)**
• **KIM, Donghoon**
  **San Jose, CA, 95134 (US)**

(74) Representative: **Kuhnen & Wacker**
**Patent- und Rechtsanwaltsbüro PartG mbB**
**Prinz-Ludwig-Straße 40A**
**85354 Freising (DE)**

(54) **MODULAR PIPELINE FOR HIGH-FIDELITY HAND-ARM MOTION SYNTHESIS AND MULTI-VIEW RENDERING**

(57)    A computer-implemented method of generating a synthetic dataset of hand and arm gestures includes generating (110), from a first conditional variational autoencoder comprising a first latent space and a first transformer decoder, a set of finger poses; generating (120), from a second conditional variational autoencoder comprising a second latent space and a second transformer decoder, a set of wrist motions; and combining (130) the set of finger poses and the set of wrist motions to generate the synthetic dataset of hand and arm gestures.

FIG. 1

```
START

110  GENERATING A SET OF FINGER POSE
     FROM A FIRST CONDITIONAL VARIATIONAL
     AUTOENCODER(CVAE)

120  GENERATING A SET OF WRIST MOTIONS
     FROM A SECOND CVAE

130  COMBINING THE FINGER POSES AND THE
     WRIST MOTIONS TO GENERATE A SYNTHETIC
     DATASET OF HAND AND ARM GESTURES

140  TRAINING A HAND POSE ESTIMATION
     MODEL AND/OR A HAND GESTURE
     RECOGNITION MODEL UTILIZING THE HAND
     AND ARM GESTURE DATABASE

END
```

**Description**

BACKGROUND

**1. Field**

The present disclosure relates to hand gesture recognition and hand-arm mesh models.

**2. Description of the Related Art**

Hand gesture databases are important to address the research and development needs in Extended Reality (XR), Human-Computer Interaction (HCI), and other domains that require data to train and evaluate hand-related models. Synthetic hand gesture databases are less costly than 3D capturing and annotating real-world data. However, related art dynamic hand gesture datasets often constrain gestures to fixed combinations of global wrist motions and specific finger poses (i.e., a rigid definition of hand gestures and a lack of motion modularity). Some synthetic hand pipelines may focus on limited 3D hands with random poses under limited viewpoints. Accordingly, some synthetic hand datasets may lack semantically meaningful gestures, motion dynamism, and data variation. For example, in some systems, simple wrist movements like moving a fist left, right, up, or down may be treated as distinct, unrelated gestures. Such rigid definitions may fail to capture the semantic meaning, and potential variability and flexibility in hand motions. Some synthetic hand datasets may therefore lack sufficient variation in hand shapes, gestures, dynamics, and viewpoints to robustly train and test 3D hand pose estimation (HPE) and hand gesture recognition (HGR) systems.

Additionally, some hand gesture databases may lack full hand-arm dynamics (i.e., realistic coordination between forearm, wrist, and fingers may be missing from datasets). For instance, unless specifically designed for very limited 3D models, the forearms may not be dynamically aligned with the hands.

The above information disclosed in this Background section is only for enhancement of understanding of the background of the invention and therefore it may contain information that does not constitute prior art.

SUMMARY

The present disclosure relates to various embodiments of a computer\-implemented method of generating a synthetic dataset of hand and arm gestures. In one embodiment, the method includes generating, from a first conditional variational autoencoder including a first latent space and a first transformer decoder, a set of finger poses; generating, from a second conditional variational autoencoder including a second latent space and a second transformer decoder, a set of wrist motions; and combining the set of finger poses and the set of wrist motions to generate the synthetic dataset of hand and arm gestures.

The combining may include performing a Cartesian product of the set of finger poses and the set of wrist motions.

The first conditional variational autoencoder may be different than the second conditional variational autoencoder.

The first transformer decoder may have eight layers, and the second transformer decoder may have two layers.

The method may also include generating a hand mesh model of the hand and arm gestures.

The set of finger poses may include at least one number gesture, at least one trigger gesture, and at least one special gesture.

The present disclosure also relates to various embodiments of a computer-based method of generating a hand-arm mesh model. In one embodiment, the method includes generating a hand mesh model; and joining an arm mesh model to the hand mesh model. Joining the arm mesh model to the hand mesh model includes identifying wrist boundary vertices of the hand mesh model and the arm mesh model; ensuring a number of the wrist boundary vertices of the hand mesh model is equal to a number of the wrist boundary vertices of the arm mesh model; and applying a wrist rotation matrix to the hand mesh model.

The method may also include removing overlapping faces between the hand mesh model and the arm mesh model at a wrist of the hand-arm mesh model.

The method may also include interpolating between the hand mesh model and the arm mesh model at the wrist to prevent visual seams between the hand mesh model and the arm mesh model.

The method may also include applying a skin texture to the hand mesh model; and propagating the skin texture of the hand mesh model to the arm mesh model.

The hand mesh model may be a NIMBLE model.

The arm mesh model may be a SMPL-X model.

The method may include applying a global transformation to the hand-arm mesh model.

Generating the hand mesh model may include converting a MANO hand model to a NIMBLE hand model.

The hand mesh model may be a Handy model.

The present disclosure also relates to various embodiments of simulating real-world camera configurations. The method may include arranging cameras in a hemispherical configuration around a hand-arm mesh model; and capturing hand motions of the hand-arm mesh model from different perspectives with the cameras.

The cameras may include static cameras.

The cameras may include dynamic cameras.

The dynamic cameras may include first camera having a close-up lens facing a palm side of the hand-arm mesh model, and a pair of stereo cameras facing a back side of the hand-arm mesh model.

The method may also include generating the hand-arm mesh model, which may include generating, by a processor, a hand mesh model; and joining, by the processor, an arm mesh model to the hand mesh model to generate the hand-arm mesh model. Joining the arm mesh model to the hand mesh model may include identifying, by the processor, wrist boundary vertices of the hand mesh model and the arm mesh model; controlling, by the processor, a number of the wrist boundary vertices of the hand mesh model to be equal to a number of the wrist boundary vertices of the arm mesh model; and applying, by the processor, a wrist rotation matrix to the hand mesh model.

This summary is provided to introduce a selection of concepts that are further described below in the detailed description. This summary is not intended to identify key or essential features of the claimed subject matter, nor is it intended to be used in limiting the scope of the claimed subject matter. One or more of the described features may be combined with one or more other described features to provide a workable method or device.

BRIEF DESCRIPTION OF THE DRAWINGS

The features and advantages of embodiments of the present disclosure will be better understood by reference to the following detailed description when considered in conjunction with the accompanying figures. In the figures, like reference numerals are used throughout the figures to reference like features and components. The figures are not necessarily drawn to scale.

FIG. 1 is a flowchart illustrating tasks of a method of generating a high-fidelity synthetic dataset of hand and arm gestures according to one embodiment of the present disclosure;

FIG. 2 depicts a conditional variational autoencoder (CVAE) utilized to synthesize diverse three-dimensional finger gesture sequences and wrist motion sequences according to one embodiment of the present disclosure;

FIG. 3 depicts finger gestures, wrist motions, and the Cartesian product of these finger gestures and wrist motions according to one embodiment of the present disclosure;

FIG. 4 is a flowchart illustrating tasks of a "cut-and-stitch" method of generating a hand-arm mesh model according to one embodiment of the present disclosure;

FIGS. 5A-5E depict the cut-and-stitch method of generating the hand-arm mesh model according to one embodiment of the present disclosure;

FIGS. 6A-6D depict a simulation of near static cameras, a simulation of dynamic cameras, a simulation of far static cameras, and a simulation of a combination of near static cameras, dynamic cameras, and far static cameras, respectively, according to embodiments of the present disclosure;

FIGS. 7A-7B are a perspective view and a schematic block diagram, respectively, of a virtual reality and/or augmented reality (VR/AR) system according to one embodiment of the present disclosure; and

FIG. 8 is an overview of a hand-arm synthesis pipeline according to one embodiment of the present disclosure.

DETAILED DESCRIPTION

Hereinafter, example embodiments will be described in more detail with reference to the accompanying drawings, in which like reference numbers refer to like elements throughout. The present invention, however, may be embodied in various different forms, and should not be construed as being limited to only the illustrated embodiments herein. Rather, these embodiments are provided as examples so that this disclosure will be thorough and complete, and will fully convey the aspects and features of the present invention to those skilled in the art. Accordingly, processes, elements, and techniques that are not necessary to those having ordinary skill in the art for a complete understanding of the aspects and features of the present invention may not be described. Unless otherwise noted, like reference numerals denote like elements throughout the attached drawings and the written description, and thus, descriptions thereof may not be repeated.

In the drawings, the relative sizes of elements, layers, and regions may be exaggerated and/or simplified for clarity. Spatially relative terms, such as "beneath," "below," "lower," "under," "above," "upper," and the like, may be used herein for ease of explanation to describe one element or feature's relationship to another element(s) or feature(s) as illustrated in the figures. It will be understood that the spatially relative terms are intended to encompass different orientations of the device in use or in operation, in addition to the orientation depicted in the figures. For example, if the device in the figures is turned over, elements described as "below" or "beneath" or "under" other elements or features would then be oriented "above" the other elements or features. Thus, the example terms "below" and "under" can encompass both an orientation of above and below. The device may be otherwise oriented (e.g., rotated 90 degrees or at other orientations) and the spatially relative descriptors used herein should be interpreted accordingly.

It will be understood that, although the terms "first," "second," "third," etc., may be used herein to describe various elements, components, regions, layers and/or sections, these elements, components, regions, layers and/or sections should not be limited by these terms. These terms are used to distinguish one element, component, region, layer or section from another element, component, region, layer or section. Thus, a first element, component, region, layer or section described below could be termed a second element, component, region, layer or section, without departing from the scope of the present invention.

It will be understood that when an element or layer is referred to as being "on," "connected to," or "coupled to" another element or layer, it can be directly on, connected to, or coupled to the other element or layer, or one or more intervening elements or layers may be present. In addition, it will also be understood that when an element or layer is referred to as being "between" two elements or layers, it can be the only element or layer between the two elements or layers, or one or more intervening elements or layers may also be present.

The terminology used herein is for the purpose of describing particular embodiments and is not intended to be limiting of the present invention. As used herein, the singular forms "a" and "an" are intended to include the plural forms as well, unless the context clearly indicates otherwise. It will be further understood that the terms "comprises," "comprising," "includes," and "including," when used in this specification, specify the presence of the stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Expressions such as "at least one of," when preceding a list of elements, modify the entire list of elements and do not modify the individual elements of the list.

As used herein, the term "substantially," "about," and similar terms are used as terms of approximation and not as terms of degree, and are intended to account for the inherent variations in measured or calculated values that would be recognized by those of ordinary skill in the art. Further, the use of "may" when describing embodiments of the present invention refers to "one or more embodiments of the present invention." As used herein, the terms "use," "using," and "used" may be considered synonymous with the terms "utilize," "utilizing," and "utilized," respectively. Also, the term "exemplary" is intended to refer to an example or illustration.

Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and/or the present specification, and should not be interpreted in an idealized or overly formal sense, unless expressly so defined herein.

The present disclosure relates to various embodiments of a method of generating a high-fidelity synthetic dataset of hand and arm gestures utilizing a phase-aware conditional variational autoencoder (CVAE) framework. The dataset of hand and arm gestures may be utilized in applications such as Hand Pose Examination (HPE), Hand Gesture Recognition (HGR), Extended Reality (XR), Human-Computer Interaction (HCI), or other domains that require realistic synthetic data to train and evaluate hand-related models. Generating and utilizing a synthetic hand gesture dataset that includes semantically meaningful gestures, motion dynamism, and data variation is configured to improve the training of hand-related models (e.g., hand-related models utilized in HPE, HGR, XR, or HCI applications) and the generation of a synthetic hand gesture dataset is less costly than 3D capturing and annotating real-world data (e.g., utilizing an array of cameras to capture real-world hand poses and then annotating those poses before utilizing the captured images to train a hand-related model). In one or more embodiments, the synthetic dataset generation is split into two CVAE streams (i.e., a dual-CVAE architecture), one for finger gestures (i.e., local gestures) and another for wrist motions (i.e., global motions) that are combined via a Cartesian product to create a diverse and flexible hand gesture database that includes gesture categories beyond those in existing related art datasets.

The present disclosure also relates to various embodiments of a cut-and-stitch method of generating a hand-arm mesh model by stitching an arm mesh template (e.g., a SMPL-X arm mesh model) to a NIMBLE hand mesh model. In one or more embodiments, the cut-and-stitch method is configured to enable dynamic hand articulation while keeping the arm's attachment stable. Additionally, in one or more embodiments, the cut-and-stitch method is configured to minimize (or at least reduce) visual seams between the hand mesh model and the arm model and to propagate the skin texture/tone of the hand model to the arm to ensure a uniform (or substantially uniform) skin tone between the hand and the arm and thereby maintain realism.

The present disclosure also relates to various embodiments of simulating a real-world setup of cameras in a hemispherical configuration around a hand-arm mesh model. The cameras are configured to capture diverse perspectives of the hand gestures articulated by the hand-arm mesh model. The cameras may include static cameras and/or dynamic cameras.

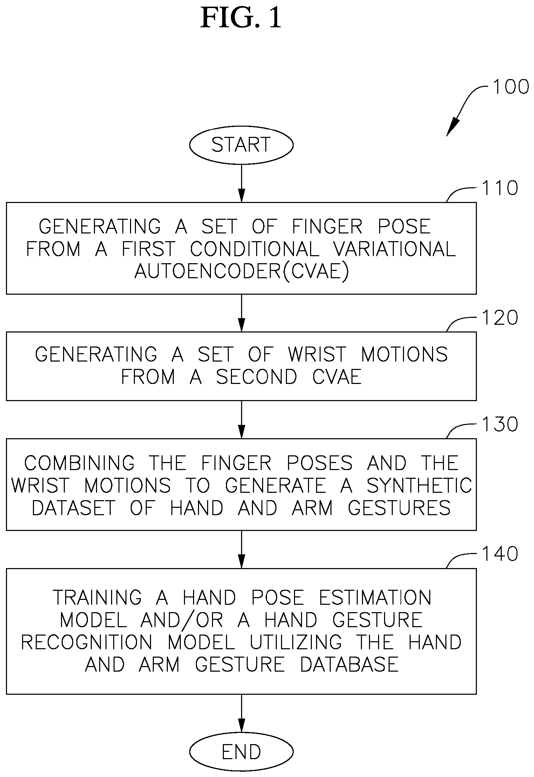

FIG. 1 is a flowchart illustrating aspects of a method **100** of generating a high-fidelity synthetic dataset of hand and arm gestures according to one embodiment of the present disclosure. Although FIG. 1 illustrates various operations in a method of generating a high-fidelity synthetic dataset of hand and arm gestures according to some embodiments, embodiments according to the present disclosure are not limited thereto. For example, according to various embodiments, the method may include additional operations, or fewer operations, or the order of operations may vary, unless otherwise stated or implied, without departing from the scope of embodiments according to the present disclosure.

As illustrated and described below, embodiments according to the present disclosure may utilize a dual conditional variation autoencoder (CVAE), that separately models global wrist motions and local finger gestures, that may be combined to create relatively diverse and flexible hand gestures. For example, in the illustrated embodiment, the method **100** includes a task **110** of generating a set of finger poses (e.g., finger poses that represent semantic meaning, such as an extended index finger to represent the number "1" or a closed thumb and index finger in a circle representing "ok"). In one or more embodiments, the task **110** of generating the finger poses utilizes a first conditional variational autoencoder (CVAE). CVAEs are unsupervised generative models that are configured to generate samples from an input by encoding the input data into a latent representation and then reconstructing the input from the latent space. CVAEs extend variational autoencoders (VAEs) by incorporating conditional information, such as class labels, during training and inference, which enables for the controlled generation of data based on specific attributes or labels.

Additionally, in the illustrated embodiment, the method **100** also includes a task **120** of generating a set of wrist motions. These wrist motions are indicative or representative of global hand motions. In one or more embodiments, the task **120** of generating the wrist motions utilizes a second CVAE. The second CVAE may be different than the first CVAE utilized in task **110** to generate the set of finger poses.

Thus, in contrast to some systems or datasets, that may constrain gestures to fixed combinations of global wrist motions and specific finger poses, embodiments according to the present disclosure may be capable of separately modeling global wrist motions and local finger gestures, as shown in task 110 and 120 and discussed in more detail below.

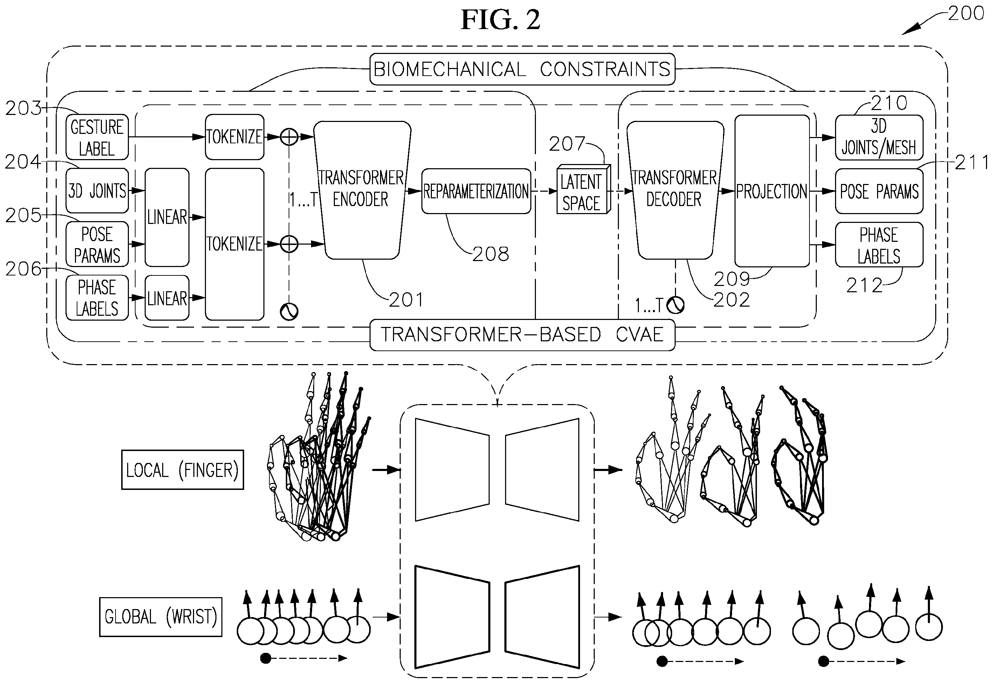

FIG. 2 is a schematic representation of a CVAE **200** utilized in tasks **110** and **120**. The left side of the diagram in FIG. 2 depicts the CVAE **200** during training and the right side of the diagram in FIG. 2 depicts the CVAE **200** during inference (e.g., generation of the finger poses or the wrist motions). As illustrated in FIG. 2, the CVAE **200** includes a transformer encoder **201** and a transformer decoder 202. During training of the CVAE **200**, gesture labels **203**, 3D joints **204**, pose parameters **205**, and phase labels **206** are linearized and tokenized and then input into the transformer encoder **201**, which encodes the input data and outputs parameters of a probability distribution (e.g., the mean and variance of a Gaussian distribution) into a latent space **207**, which is a lower-dimensional, continuous space where the input data is encoded. The pose parameters **205** refer to the configuration of the fingers (e.g., index finger pointing; thumb up; ok shape) and the phase labels **206** refer to the extent to which the finger configuration has transitioned into the final finger pose (e.g., initial position; transitioning; or final position). The transformer decoder **202** is configured to create new data that resembles the input data by sampling (e.g., utilizing a reparameterization gradient estimator **208** (also known as the reparameterization trick)) from the distribution in the latent space 207. In one or more embodiments, the CVAE **200** utilized in task **110** and/or task **120** may be the same as or similar to the CVAE described in U.S. Provisional Application No. 63/707,422, the entire contents of which are incorporated herein by reference. In particular, the disclosure of the CVAE as described in U.S. Provisional Application No. 63/707,422 is incorporated herein by reference.

In one or more embodiments, the transformer decoder **202** of the first CVAE utilized in task **110** to generate the finger poses has more layers than the transformer decoder **202** of the second CVAE **200** utilized in task **120** to generate the wrist motions. For instance, in one or more embodiments, the transformer decoder **202** of the first CVAE utilized in task **110** has eight (8) layers and the transformer decoder **202** of the second CVAE utilized in task **120** has two (2) layers.

During task **110**, inference of the first CVAE **200** is performed by inputting a text-based finger gesture label **203** (e.g., "finger pinch"; "finger swipe"; or "finger snap") into the first CVAE **200**. The latent space **207** is then randomly sampled and this sample is input into the transformer decoder **202**. The transformer decoder **202** generates a projection **209** from the sample from the latent space **207** and then the projection **209** outputs three-dimensional joints **210**, pose parameters **211**, and phase labels **212**. In one or more embodiments, the output is a skeleton of joints in a configuration corresponding to the input finger gesture label (e.g., a skeleton of finger joints with the thumb and the index fingertips touching each other in response to the input finger gesture label being "finger pinch"). In this manner, the inference process in task **110** is configured to synthesize diverse three-dimensional finger gesture sequences (i.e., by sampling from the latent space, a variety of different 3D joints/mesh corresponding to the input text-based finger gesture label are generated).

During task **120**, inference of the second CVAE **200** is performed by inputting a text-based global (wrist) gesture label (e.g., "circle," "cross," or "upward movement") into the second CVAE **200. The** latent space **207** is then randomly sampled and this sample is then input into the transformer decoder **202**. The transformer decoder **202** generates a projection from the sample from the latent space **207** and then **the** projection outputs three-dimensional joints/mesh **210**, pose parameters **211**, and phase labels **212**. In this manner, the inference process in task **120** is configured to synthesize diverse wrist gesture sequences (i.e., by sampling from the latent space, a variety of different 3D joints/mesh corresponding to the input text-based wrist gesture label are generated).

With reference again to the embodiment illustrated in FIG. 1, the method **100** also includes a task **130** of combining the set of finger poses generated in task **110** with the set of wrist motions generated in task **120** to generate the synthetic dataset of hand and arm gestures. Accordingly, the finger gestures and the global wrist motions are synthesized separately in tasks **110** and **120** and then combined. Together, the dual process streams (i.e., the finger gestures generated from the first CVAE and the wrist motions generated from the second CVAE) generate diverse three-dimensional hand gesture sequences (i.e., diverse finger and wrist combinations are integrated to form a dataset of diverse hand gestures). In this manner, combining the two streams generates a wide range of meaningful hand gestures that extends beyond alternative datasets that may have rigid definitions or constrained combinations of hand poses, gestures, or movement trajectories. That is, the dataset of diverse hand gestures generated according to embodiments of the present disclosure provide an improvement over related art datasets that have limited hand gestures, and these enriched datasets represent a broad spectrum of gesture classes.

In one or more embodiments, the method **100** may include a task **140** of utilizing the hand and arm gesture database generated in task **130** to train a hand pose estimation model (e.g., Mobile-StereoHPE) and/or to train a hand gesture recognition model (e.g., Fast-DNN). In one or more embodiments, the trained models (e.g., the trained hand pose estimation model and/or the trained hand gesture recognition model) may be incorporated in an extended reality (XR) device, such as an augmented reality (AR) device, a virtual reality (VR) device, or a mixed reality device. In one or more embodiments, the hand and arm gesture database generated in task **130** may be utilized in a synthetic saliency-bokeh video dataset for cinematic video project.

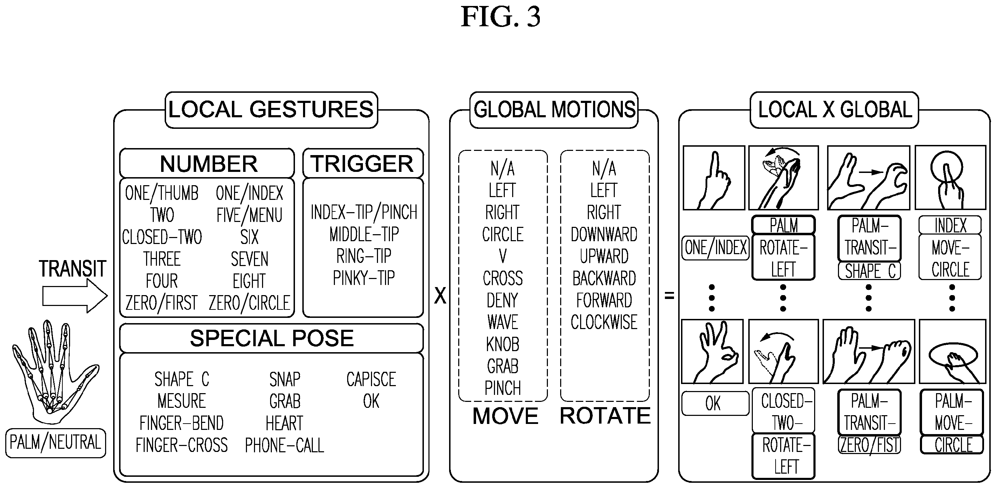

FIG. 3 depicts a set of local gestures (i.e., finger gestures), including finger gestures representing a number (e.g., the thumb or index finger extending to represent the number one, two digits extending to represent the number two, etc.), finger trigger gestures (e.g., tips of the thumb and index finger touching, tips of the thumb and middle finger touching, tips of the thumb and ring finger touching, or tips of the thumb and pinky finger touching), and finger special poses (e.g., a snap, a heart shape, a phone-call gesture, an OK gesture, etc.), and global wrist motions, such as left movement, right movement, a circular movement, a cross-shaped movement, forward movement, backward movement, upward movement, downward movement, etc. The right side of FIG. 3 depicts the Cartesian product of these finger gestures and the wrist motions, such as an index finger pointing and the wrist moving in a circle, fingers closing into a fist and sliding (translating) to the right, an open palm rotating to the left, an open palm moving in a circulation motion, etc.

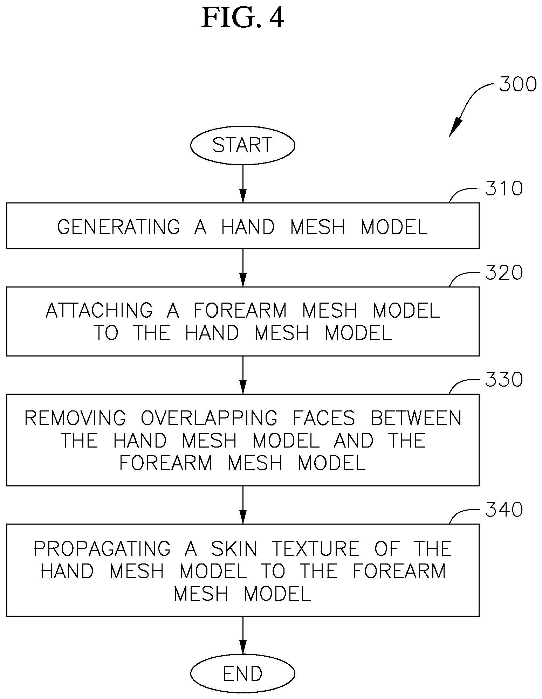

FIG. 4 is a flowchart illustrating tasks of a "cut-and-stitch" method **300** of generating a hand-arm mesh model according to one embodiment of the present disclosure. Although FIG. 4 illustrates various operations in a method of generating a hand-arm mesh model according to some embodiments, embodiments according to the present disclosure are not limited thereto. For example, according to various embodiments, the method may include additional operations, or fewer operations, or the order of operations may vary, unless otherwise stated or implied, without departing from the scope of embodiments according to the present disclosure.

In the illustrated embodiment, the method **300** includes a task **310** of generating a hand mesh model. In one or more embodiments, in task **310,** the output from the first CVAE (i.e., the skeleton of joints in various finger poses) may be transformed into a three-dimensional hand mesh utilizing MANO (hand Model with Articulated and Non-rigid defOrmations), which is a parameterized hand model. The MANO hand model is described in Romero et al, "Embodied Hands: Modeling and Capturing Hands and Bodies Together," arXiv:2201.02610 [cs.GR] (January 7, 2022), the entire content of which is incorporated herein by reference.

The MANO hand model includes a kinematic tree with 16 joints and the rotation of each joint is represented in axis-angle format aligned with a wrist-orthogonal basis. In one or more embodiments, the method may utilize an anatomically constrained version of the MANO hand model, A-MANO, to achieve anatomically accurate hand meshes. The A-MANO hand model redefines the canonical hand pose to calculate the twist, spread, and bend axes in an anatomically aligned orthogonal basis. The A-MANO hand model is described in Yang et al., "CPF: Learning a Contact Potential Field to Model the Hand-Object Interaction," In International Conference on Computer Vision (ICCV), 2021, the entire contents of which are incorporated herein by reference. The formulation of MANO can be described according to Equation 1 as follows:

$$\mathrm{M}(\theta_{m}f, \beta_{m}) = \mathcal{M}_{\not{f}}\big(\mathcal{C}(\theta_{m}c, \beta_{m})\big) \quad \textbf{(Equation 1)}$$

where $\mathcal{C}$ denotes the composition function that aligns joint angles with anatomical axes, $\mathcal{M}_{\not{f}}$ represents the flat-hand layer in MANO, and $(\theta_{m}c, \beta_{m})$ are the joint angles and shape parameters, respectively, in the anatomically aligned space.

In one or more embodiments, the MANO hand mesh is then converted into a NIMBLE hand mesh, which is a high-resolution non-rigid parametric hand model that includes bones, muscles, and skin texture. The NIMBLE hand model is described in Li et al., "NIMBLE: A Non-rigid Hand Model with Bones and Muscles," arXiv:2202.04533 [cs.CV] (July 18, 2022), the entire contents of which are incorporated herein by reference. The NIMBLE hand model provides greater variability in hand shapes and textures compared to the MANO hand model.

The NIMBLE model extends the MANO hand mesh by incorporating both geometry and appearance modeling as shown in Equation 2 as follows:

$$N(\theta_n, \beta_n, \alpha) = \mathcal{G}(\theta_n, \beta_n), \mathcal{A}(\alpha) \qquad \textbf{(Equation 2)}$$

where $\mathcal{G}$ models the hand geometry, and $\mathcal{A}$ captures the hand's textural appearance. The parameters $(\theta_n, \beta_n, \alpha)$ correspond to pose, shape, and appearance, respectively.

In one or more embodiments, the task of converting the MANO hand mesh to the NIMBLE hand mesh includes the NIMBLE model deterministically subsampling to the MANO mesh using function $\mathcal{S}$ from 5990 to 778 vertices, as shown in Equation 3 below:

$$M_v = \mathcal{S}\big(\mathcal{G}(\theta_n, \beta_n)\big),$$
$$\mathcal{S}: R^{5990 \times 3} \to R^{778 \times 3} \qquad \textbf{(Equation 3)}$$

where $M_v$ represents the MANO vertices derived from the NIMBLE vertices. Additionally, in one or more embodiments, the task of converting the MANO hand mesh to the NIMBLE hand mesh includes utilizes a gradient-based optimization method to fit the MANO pose parameter $\theta_m$ and shape parameters $\beta_m$ by minimizing the deviation between the generated MANO mesh $M(\theta_m, \beta_m)$ and the subsampled NIMBLE mesh $(M_v)$. Additionally, in one or more embodiments, to ensure plausible results, the method incorporates additional regularization terms for pose and shape parameters, as shown in Equation 4 below:

$$\theta_m^*, \beta_m^* = \arg \min_{\theta_m, \beta_m} |M_v - M(\theta_m, \beta_m)|^2 + \lambda_\theta \mathcal{R}_\theta(\theta_m) + \lambda_\beta \mathcal{R}_\beta(\beta_m) \qquad \textbf{(Equation 4)}$$

The objective function is formulated as:

$$E = \left\| M_v - M(\theta_m, \beta_m) \right\|^2 \quad \textbf{(Equation 5)}$$

where E is the reconstruction error based on L2-norm, measuring the vertex-wise distance between the subsampled MANO vertices and the optimized MANO mesh.

In the reverse direction, the MANO mesh is converted and aligned to the NIMBLE by optimizing NIMBLE's parameters and wrist translation to ensure consistency.

Although in one or more embodiments the method utilizes the MANO and NIMBLE hand models, in one or more embodiments, the method may utilize any other suitable parametric hand models, such as Handy, to achieve similar high-fidelity hand reconstruction. The Handy hand model is described in Potamias et al., "Handy: Towards a high fidelity 3D hand shape and appearance model," Proceedings of the IEEE/CVF Conference on Computer Vision and Pattern Recognition (CVPR) (June 2023), pages 4670-4680, the entire contents of which are incorporated herein by reference.

The NIMBLE hand mesh ends at the wrist, which negatively affects the realism of the generated synthetic hand models. Accordingly, in one or more embodiments, the method 300 includes a task 320 of combining the hand mesh model (e.g., the NIMBLE hand mesh model) with a forearm template model (e.g., an arm template model) to generate a complete hand-arm mesh model.

The forearm template model may be any suitable arm model, such as a SMPL-X hand-arm model that has been spliced at the wrist (i.e., cut at the wrist). The SMPL-X hand-arm model is described in Pavlakos et al., "Expressive Body Capture: 3D Hands, Face, and Body from a Single Image," the entire contents of which are incorporated herein by reference. In one or more embodiments, the forearm mesh may be an up-sampled (e.g., high-resolution) version of the SMPL-X forearm model.

In one or more embodiments, the task 320 of attaching the forearm mesh model to the hand mesh model includes identifying the wrist boundary vertices $V_w^{NIM}$ of the NIMBLE hand mesh and setting the wrist boundary vertices of the forearm model $V_w^{ARM}$ equal to the wrist boundary vertices $V_w^{NIM}$ of the NIMBLE hand mesh, as shown in Equation 6 below:

$$|V_w^{ARM}| = |V_w^{NIM}| \qquad \textbf{(Equation 6)}$$

In one or more embodiments, the task **320** of attaching the forearm mesh model to the hand mesh model is configured to maintain flexible wrist rotation of the hand-arm model while ensuring the proper alignment between the hand mesh model and the forearm mesh model. In one or more embodiments, the task **320** includes applying a wrist rotation matrix $R_w$ to the hand mesh model. The wrist rotation matrix $R_w$ is a matrix (e.g., a 3 x 3 matrix) that represents the orientation of the wrist relative to a reference frame. The wrist rotation matrix $R_w$ may be calculated utilizing Euler angles or other angular rotation representations. In one or more embodiments, the vertices $V_{stitch}$ at the stitch between the hand mesh model and the forearm mesh model are a function of a wrist rotation matrix $R_w$ that enables dynamic hand articulation of the hand-arm mesh model and stable attachment between the hand mesh model and the forearm mesh model, as shown in Equation 7 below:

$$V_{stitch} = R_w \cdot (V_w^{NIM} - C_w) + C_w + V^{SMP} \qquad \textbf{(Equation 7)}$$

Additionally, in one or more embodiments, the task **320** of attaching the forearm mesh model to the hand mesh model includes applying a global transformation ($R_g$, $t_g$) to the entire hand-and-arm mesh model as shown in Equation 8 below:

$$V^{final} = R_g \cdot V^{stitch} + t_g \qquad \textbf{(Equation 8)}$$

In one or more embodiments, the method **300** also includes a task **330** of removing overlapping faces between the hand mesh model and the forearm mesh model at the wrist.

In one or more embodiments, the "Cut-and-Stitch" method **300** also includes a task **340** of propagating a skin texture of the hand mesh model to the forearm to ensure uniform skin tone and to maintain realism. In one or more embodiments, the task **340** includes UV mapping and blending textures for a consistent (or substantially consistent) skin texture across the hand and the arm. UV mapping is the process of unwrapping or unfolding a three-dimensional model into a two-dimensional space, thereby allowing for the application of textures to the surface of the model (i.e., a UV map is a vertex map that stores horizontal (U) and vertical (V) positions on a two-dimensional texture map). In one or more embodiments, in task **340,** the UV mapping at the wrist is adjusted by interpolating between the hand mesh model (e.g., the NIMBLE hand model) and the arm mesh model (e.g., SMPL-X arm model or the upsampled SMPL-X arm model) to prevent visual seams between the hand mesh model and the forearm mesh model.

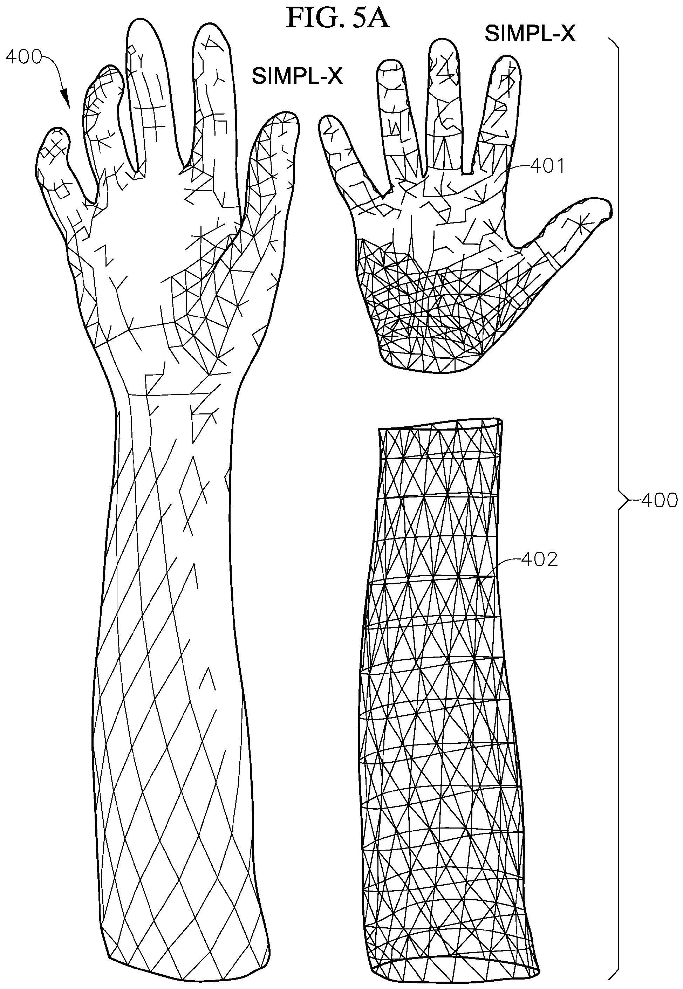

FIGS. 5A-5E depict further details of aspects of the cut-and-stitch method **300** of generating the hand-arm mesh model according to one embodiment of the present disclosure. As illustrated and described, the method of generating the hand-arm mesh model may cut or divide portions of the hand and arm into different segments in order to enable flexible movement and consistent skin tone between the different segments.

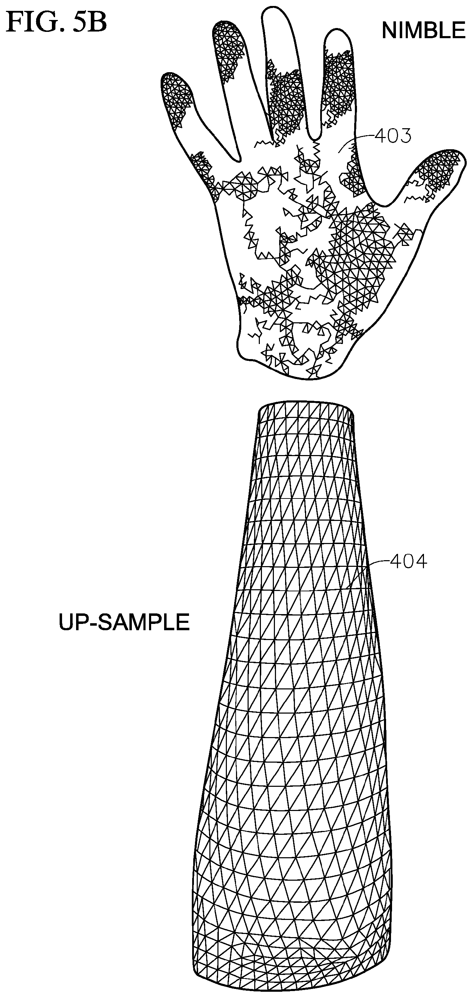

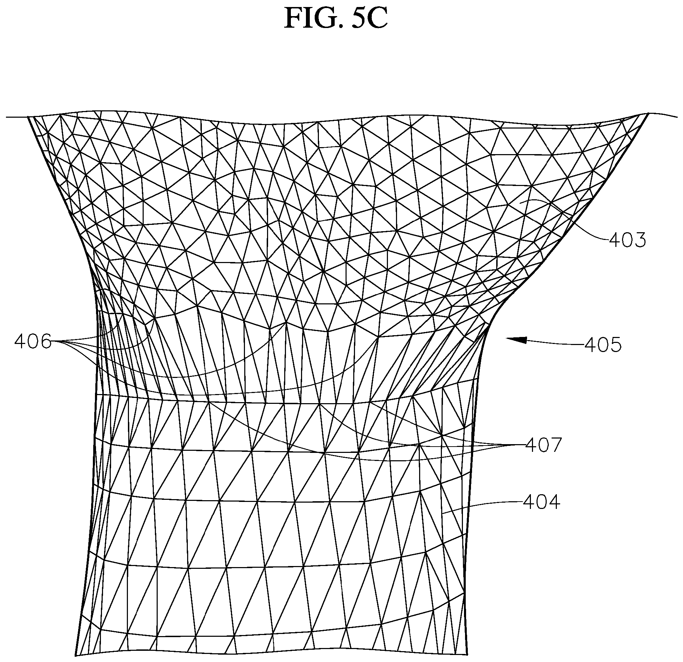

FIG. 5A depicts a hand-arm mesh model **400** (e.g., comprising a SMPL-X forearm mesh model) that has been "cut" (sectioned or segmented) at the wrist (i.e., a parametric arm model **400** that has been sectioned or divided between the hand portion **401** and the forearm portion **402).** FIG. 5B depicts a hand mesh model **403** (e.g., a NIMBLE hand mesh model) and an up-sampled (e.g., higher resolution) version **404** of the forearm mesh model **402** illustrated in FIG. 5A. In one or more embodiments, FIG. 5B depicts the task **310** of generating the hand mesh model. FIG. 5C depicts the hand mesh model **403** and the forearm mesh model **404** (e.g., the up-sampled SMPL-X forearm model) attached ("stitched") together at the wrist **405.** In one or more embodiments, FIG. 5C depicts the task **320** of combining a hand mesh model with a forearm template model to generate a complete hand-arm mesh model. In one or more embodiments, FIG. 5C also depicts the task **330** of removing overlapping faces between the hand mesh model and the forearm mesh model at the wrist. As described above, the process of joining the hand mesh model **403** to the forearm mesh model **404** includes identifying the wrist boundary vertices **406** of the hand mesh model **403** and setting the wrist boundary vertices **407** of the forearm mesh model **404** equal to the wrist boundary vertices **406** of the hand mesh model **403** according to Equation 6 above. Additionally, as described above, the process of joining the hand mesh model **403** to the forearm mesh model **404** also includes applying a wrist rotation matrix $R_w$ that enables dynamic hand articulation of the hand-arm mesh model and stable attachment between the hand mesh model **403** and the forearm mesh model **404** according to Equation 7 above, and applying a global transformation according to Equation 8 above.

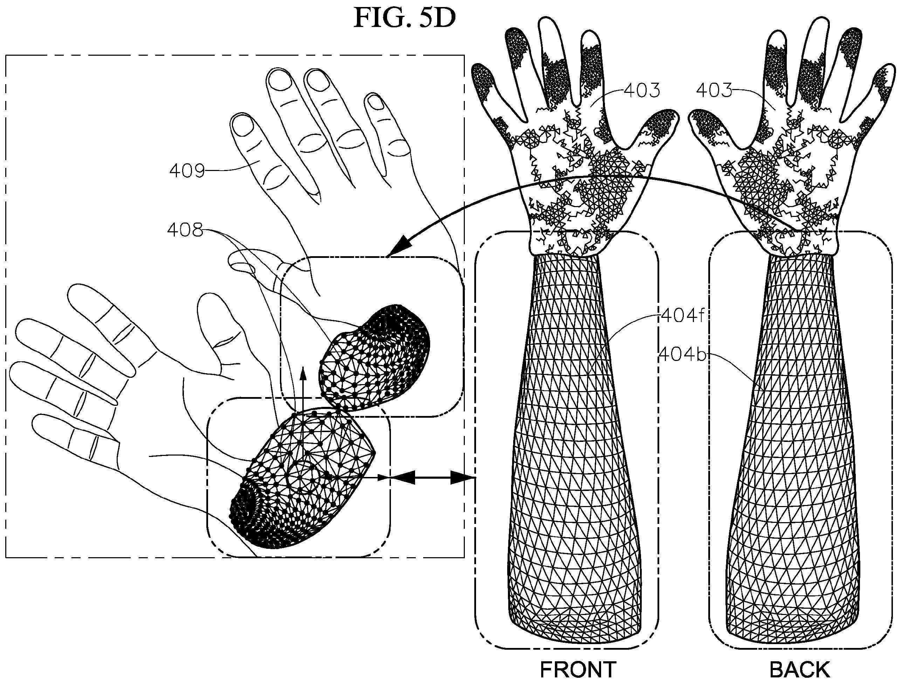

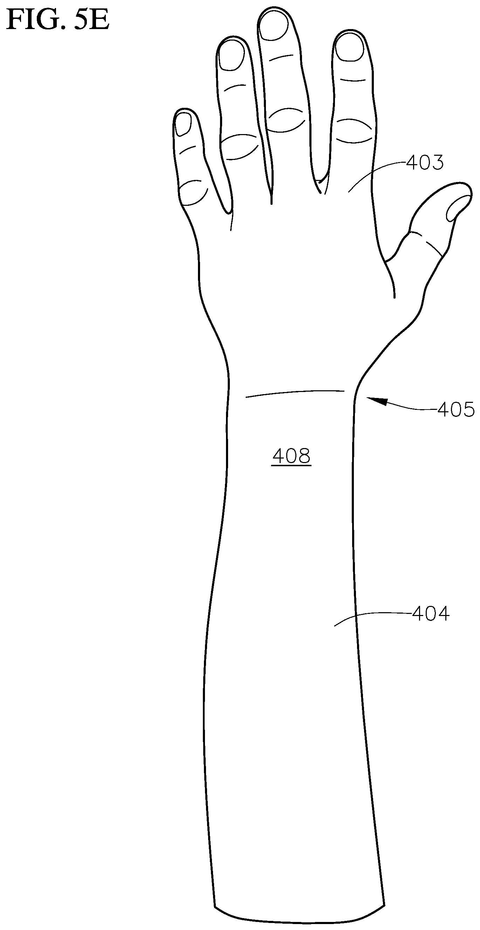

FIG. 5D depicts the selection of a texture **408** (e.g., a skin texture and/or skin tone) for the front **404$_f$** and the back **404_b** of the forearm mesh model **404** from a hand mesh texture **409** (e.g., the skin texture and/or skin tone for the forearm mesh model **404** is selected from the UV skin texture map **409** for the NIMBLE hand mesh model **403).** FIG. 5E depicts the skin texture and/or skin tone **408** being applied to both the hand mesh model **403** and the forearm mesh model **404.** In one or more embodiments, FIGS. 5D and 5E depict the task **340** of propagating a skin texture of the hand mesh model **403** to the forearm mesh model **404** to ensure uniform skin tone and to maintain realism of the hand-arm mesh model.

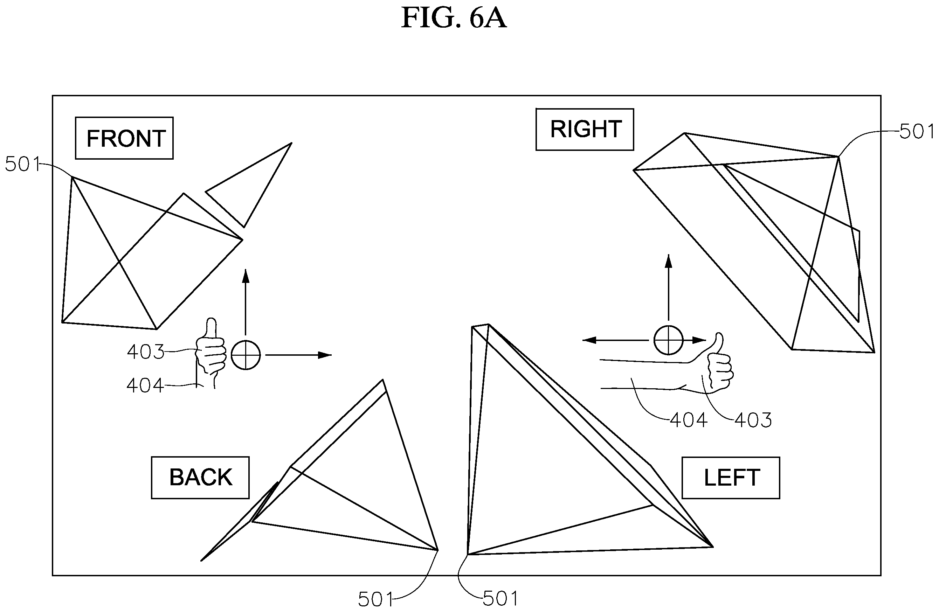

FIGS. 6A-6D depict simulations of real-world camera setups in a hemispherical configuration or arrangement that are configured to capture hand motions (including intricate finger movements and global wrist motions) of a hand-arm mesh model (e.g., the hand-arm mesh model depicted in FIGS. 5A-5E and formed according to the cut-and-stitch method **300** depicted in FIG. 4). These camera configurations support rendering from various camera viewpoints (e.g., mono/-stereo, egocentric/all-centric, static/dynamic), which enhances robustness for real-world scenarios.

FIG. 6A depicts static cameras **501** with standard lenses and ultra-wide-angle lenses positioned at front, back, side, and diagonal positions around a hand-arm mesh model and relatively close to the hand-arm mesh model. In one or more embodiments, the static cameras **501** may include four cameras (positioned to the front, back, left, and right of the hand-arm mesh model) each having a focal length of 35 mm, a sensor width (W) and height (H) in millimeters (mm) of 36.0 x 24.0, and image size of 1200 x 800, and an aperture of f/1.8. In one or more embodiments, the static cameras **501** may also include two cameras (positioned at the front-right and the front-left diagonal positions with respect to the hand-arm mesh model) each having a focal length of 18 mm, a sensor width (W) and height (H) in millimeters (mm) of 22.3 x 14.9, and image size of 1200 x 800, and an aperture of f/2.8. In one or more embodiments, the static cameras **501** may also include two ultra-wide-angle cameras (positioned at the front-right and the front-left diagonal positions with respect to the hand-arm mesh model) each having a focal length of 13 mm, a sensor width (W) and height (H) in millimeters (mm) of 6.17 x 4.55, and image size of 3200 x 2400, and an aperture of f/2.2. The static cameras **501** depicted in FIG. 6A are positioned at key angles that are configured to capture comprehensive and distortion-free coverage of the motions of the hand-arm mesh model.

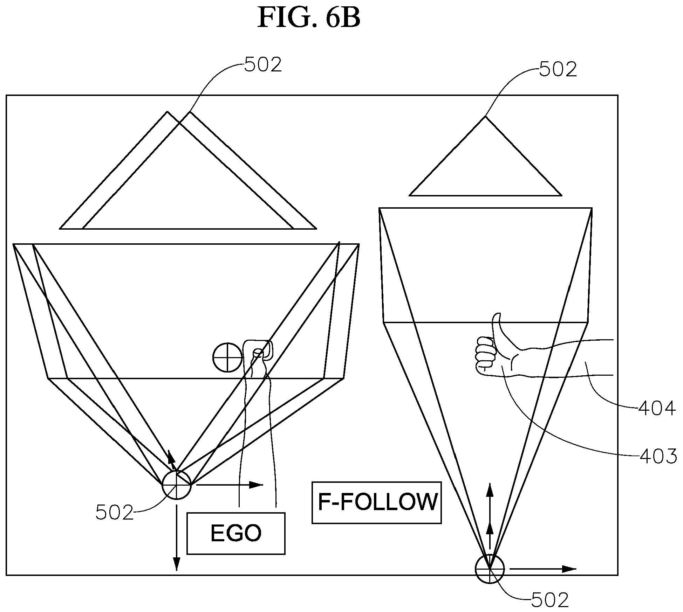

FIG. 6B depicts dynamic cameras **502,** including a close-up lens positioned on the palm side of the hand-arm mesh model and configured to capture intricate finger movements of the hand-arm mesh model and stereo pair of lenses positioned on the back side of the hand-arm mesh model configured to track the movement and three-dimensional (3D) depth of the back of the hand-arm mesh mode. In one or more embodiments, the dynamic cameras **502** may include one camera (positioned at the palm side of the hand-arm mesh model) having a focal length of 85 mm, a sensor width (W) and height (H) in millimeters (mm) of 36.0 x 24.0, an image size of 1200 x 800, and an aperture of f/1.4, and a stereo pair of cameras (positioned at the back side of the hand-arm mesh model) having a focal length of 18 mm, a sensor width (W) and height (H) in millimeters (mm) of 27.36 x 24.0, and image size of 1200 x 800, and an aperture of f/2. In one or more embodiments, the dynamic cameras **502** may include one camera (positioned at the palm side of the hand-arm mesh model) having a focal length in a range from 70 mm to 100 mm, a sensor width (W) and height (H) in millimeters (mm) in a range from 24 x 16 to 48 x 32, an image size of in a range from 800 x 600 to 1600 x 1000, and an aperture in a range from f/1.2 to f/1.6, and a stereo pair of cameras (positioned at the back side of the hand-arm mesh model) having a focal length of in a range from 12 mm to 24 mm, a sensor width (W) and height (H) in millimeters (mm) in a range from 18 x 16 to 36 x 32, and image size in a range from 800 x 600 to 1600 x 1000.

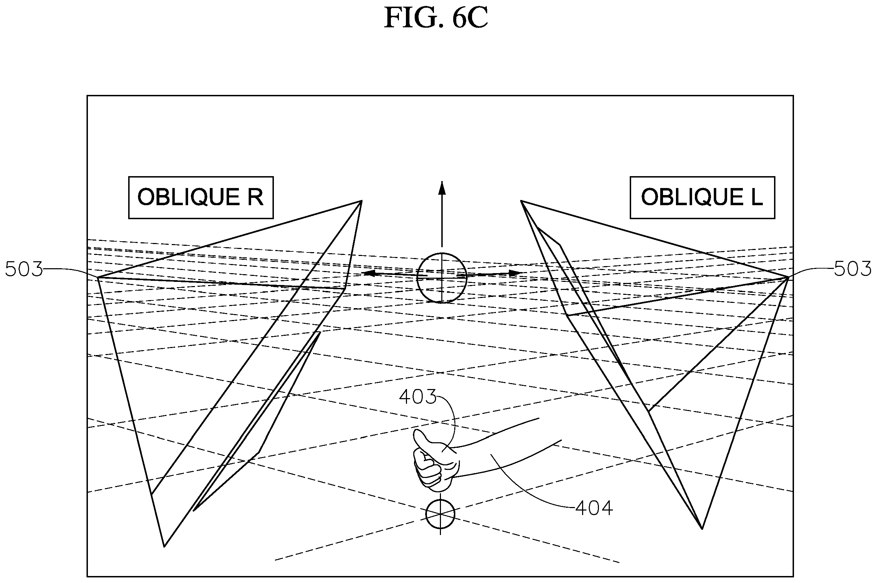

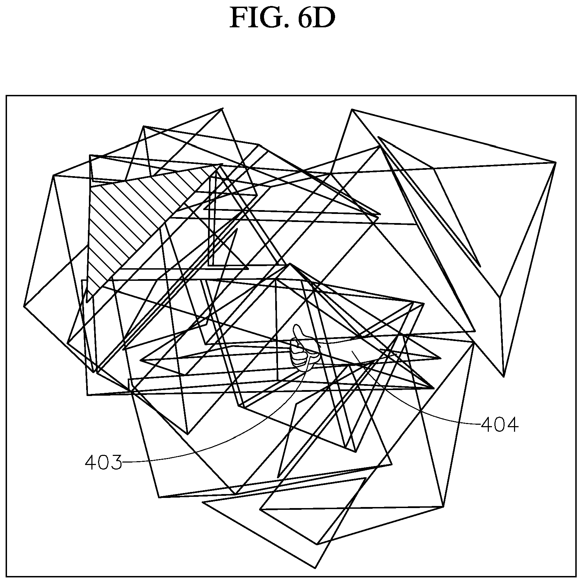

FIG. 6C depicts static cameras **503** with standard lenses and ultra-wide-angle lenses positioned at front, back, side, and diagonal positions around a hand-arm mesh model and relatively far away from the hand-arm mesh model compared to the arrangement of the cameras in FIG. 6A. FIG. 6D depicts a combined view of all of the camera setups depicted in FIGS. 6A-6C.

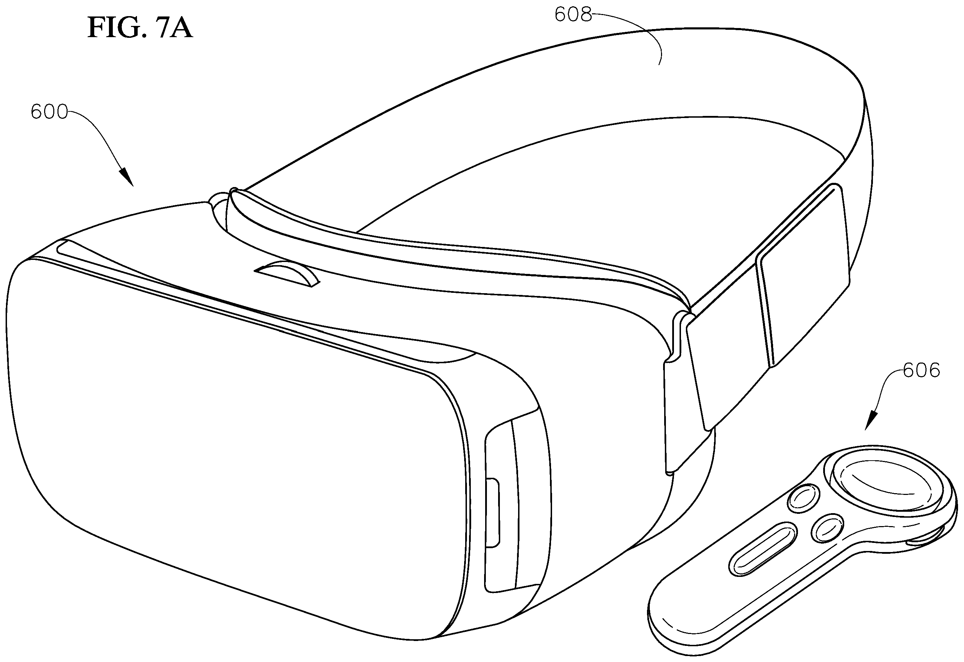

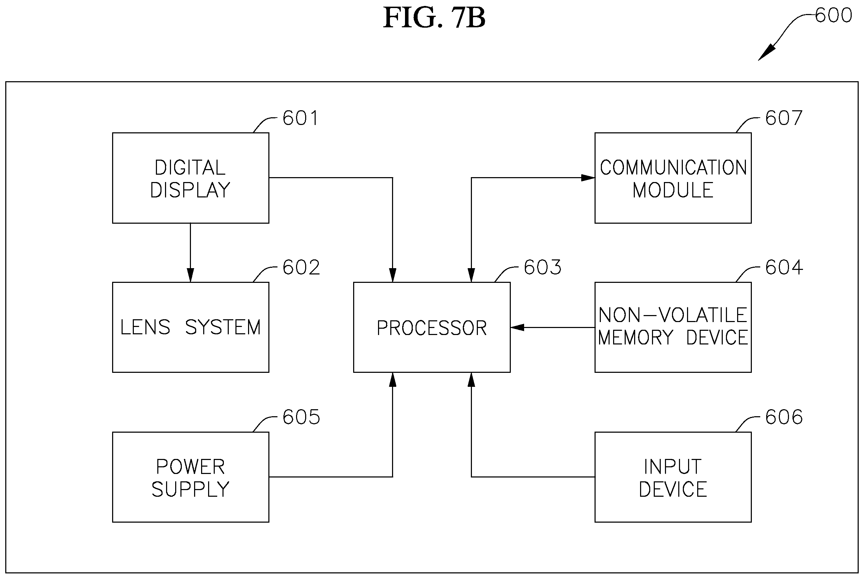

With reference now to FIGS. 7A-7B, a virtual reality and/or augmented reality (VR/AR) system **600** according to one embodiment of the present disclosure includes a digital display **601** (e.g., a digital micro-display, such as an organic light-emitting diode (OLED) display) and a lens system **602** (i.e., viewing optics) in front of the digital display **601.** When the VR/AR system **600** is worn by a user, the lens system **602** is between the digital display **601** and the user's eye.

In one or more embodiments, the VR/AR system **600** also includes a processor **603** coupled to the digital display **601,** a non-volatile memory device **604** (e.g., flash memory, ferroelectric random-access memory (F-RAM), magnetostrictive RAM (MRAM), FeFET memory, and/or resistive RAM (ReRAM) memory) coupled to the processor **603,** and a power supply **605** (e.g., one or more secondary batteries) coupled to the processor **603.** The non-volatile memory device **604** includes executable instructions (i.e., computer-readable code) which, when executed by the processor **603,** cause the processor **603** to control the display of various images by the display **601.** In one or more embodiments, the VR/AR system **600** may include an input device **606** (e.g., a handheld controller) configured to perform various operations, such as modifying the images displayed by the display **601.** In one or more embodiments, the VR/AR system **600** may include a communication module (e.g., a network adapter) **607** configured to support establishing a direct (e.g., wired) communication channel or a wireless communication channel between the VR/AR system **600** and an external electronic device (e.g., another electronic device or a server) and performing communication via the established communication channel. In one or more embodiments, the VR/AR system **600** also includes a headband or strap **608** (e.g., an adjustable band) configured to secure the VR/AR system **600** to a user's head.

The term "processor" is used herein to include any combination of hardware, firmware, and/or software, employed to process data or digital signals. The hardware of a processor may include, for example, application specific integrated circuits (ASICs), general purpose or special purpose central processors (CPUs), digital signal processors (DSPs), graphics processors (GPUs), and programmable logic devices such as field programmable gate arrays (FPGAs). In a processor, as used herein, each function is performed either by hardware configured, i.e., hard-wired, to perform that function, or by more general purpose hardware, such as a CPU, configured to execute instructions stored in a non-transitory storage medium. A processor may be fabricated on a single printed wiring board (PWB) or distributed over several interconnected PWBs. A processor may contain other processors; for example, a processor may include two processors, an FPGA and a CPU, interconnected on a PWB. The processor **603** may include a main processor (e.g., a central processing unit (CPU) or an application processor (AP)), and an auxiliary processor (e.g., a graphics processing unit (GPU), an image signal processor (ISP), a sensor hub processor, or a communication processor (CP)) that is operable independently from, or in conjunction with, the main processor. Additionally or alternatively, the auxiliary processor may be adapted to consume less power than the main processor, or execute a particular function. The auxiliary processor may be implemented as being separate from, or a part of, the main processor. The auxiliary processor may control at least some of the functions or states related to at least one component among the components of the electronic device, instead of the main processor while the main processor is in an inactive (e.g., sleep) state, or together with the main processor while the main processor is in an active state (e.g., executing an application). The auxiliary processor (e.g., an image signal processor or a communication processor) may be implemented as part of another component functionally related to the auxiliary processor.

The communication module **607** may include one or more communication processors that are operable independently from the processor **603** (e.g., the AP) and supports a direct (e.g., wired) communication or a wireless communication with another device. The communication module **607** may include a wireless communication module (e.g., a cellular communication module, a short-range wireless communication module, or a global navigation satellite system (GNSS) communication module) or a wired communication module (e.g., a local area network (LAN) communication module or a power line communication (PLC) module). A corresponding one of these communication modules may communicate with the external electronic device or a server via a short-range communication network (e.g., BLUETOOTH™, wireless-fidelity (Wi-Fi) direct, or a standard of the Infrared Data Association (IrDA)) or a long-range communication network (e.g., a cellular network, the Internet, or a computer network (e.g., LAN or wide area network (WAN)). These various types of communication modules may be implemented as a single component (e.g., a single IC), or may be implemented as multiple components (e.g., multiple ICs) that are separate from each other. The communication module **607** may identify and authenticate the VR/AR system **600** in a communication network, such as the short-range communication network or the long-range communication network, using subscriber information (e.g., international mobile subscriber identity (IMSI)) stored in a subscriber identification module. In one or more embodiments, the communication module 507 may include an antenna configured to transmit or receive a signal and/or power to or from the outside (e.g., an external electronic device) of the VR/AR system **600**. In one or more embodiments, the communication module **607** may include one or more antennas, and, therefrom, at least one antenna appropriate for a communication scheme used in the communication network, such as the long-range or the short-range communication network, may be selected, for example, by the communication module **607**. The signal or the power may then be transmitted or received between the communication module **607** and the external electronic device via the selected at least one antenna.

Commands or data may be transmitted or received between the VR/AR system **600** and an external electronic device via a server coupled with a long-range communication network. Each of the electronic devices may be a device of a same type as, or a different type, from the VR/AR system **600**. All or some of operations to be executed at the VR/AR system **600** be executed at one or more of the external electronic devices. For example, if the VR/AR system **600** should perform a function or a service automatically, or in response to a request from a user or another device, the VR/AR system **600,** instead of, or in addition to, executing the function or the service, may request the one or more external electronic devices to perform at least part of the function or the service. The one or more external electronic devices receiving the request may perform the at least part of the function or the service requested, or an additional function or an additional service related to the request and transfer an outcome of the performing to the VR/AR system **600**. The VR/AR system **600** may provide the outcome, with or without further processing of the outcome, as at least part of a reply to the request. To that end, a cloud computing, distributed computing, or client-server computing technology may be used, for example.

In one or more embodiments, the memory device **604** of the VR/AR system **600** may include a hand pose estimation model or module (e.g., Mobile-StereoHPE) and/or a hand gesture recognition model or module (e.g., Fast-DNN) such that the VR/AR system 500 is configured to recognize hand gestures by the user as input commands. In one or more embodiments, hand pose estimation model or module and/or the hand gesture recognition model or module may have been trained on the hand and arm gesture database generated according to embodiments of the present disclosure.

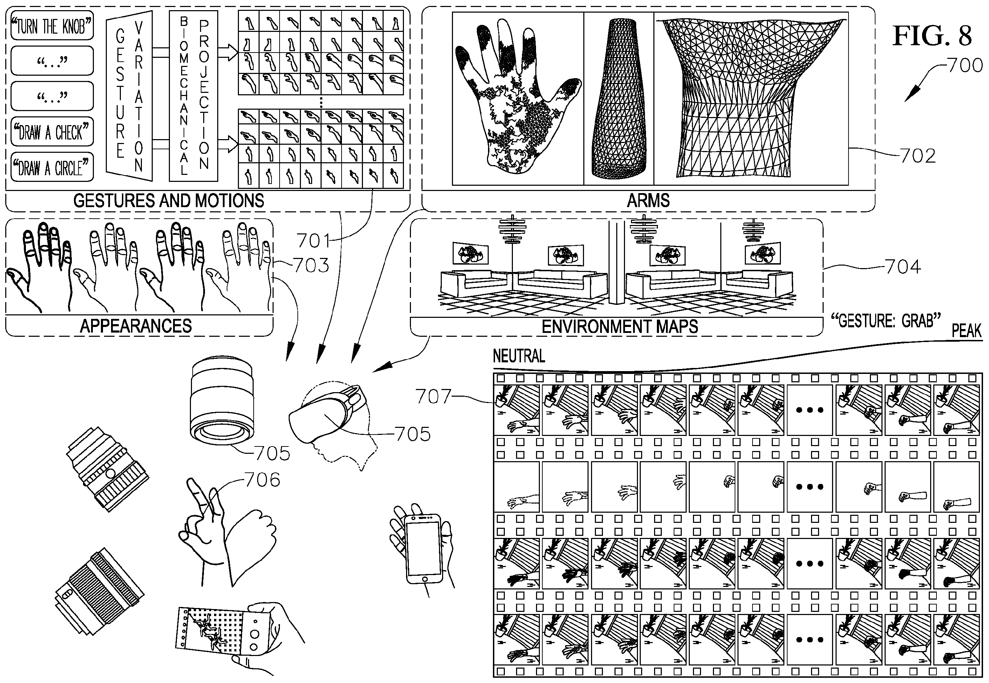

FIG. 8 is an overview of a hand-arm synthesis pipeline **700** according to one embodiment of the present disclosure. The left side of FIG. 8 depicts a database comprising a plurality of different finger gestures and hand motions **701** (e.g., pinch, swipe, grab, point gestures, etc.), hand and arm mesh models **702,** a variety of different skin textures (appearances) **703** applied to the hand and arm mesh models **702,** and environmental maps **704.** The center portion of FIG. 8 depicts a multi-camera setup **705** (e.g., camera lenses, AR/VR headsets, smartphones, etc.) surrounding a hand and arm mesh model **706** forming a semantic gesture (e.g., the index and middle fingers extended to represent the number 2). The right side of FIG. 8 depicts rendered RGBD sequences **707,** which combine both color (red-green-blue (RGB)) and depth (D) information, of realistic hand gestures (e.g., a "grab" hand gesture) selected from the database of different finger gestures and hand motions **701,** dynamic lighting, and diverse environments selected from the environment maps **704.**

The electronic or electric devices and/or any other relevant devices or components according to embodiments of the present invention described herein may be implemented utilizing any suitable hardware, firmware (e.g. an application-specific integrated circuit), software, or a combination of software, firmware, and hardware. For example, the various components of these devices may be formed on one integrated circuit (IC) chip or on separate IC chips. Further, the various components of these devices may be implemented on a flexible printed circuit film, a tape carrier package (TCP), a printed circuit board (PCB), or formed on one substrate. Further, the various components of these devices may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random access memory (RAM). The computer program instructions may also be stored in other non-transitory computer readable media such as, for example, a CD-ROM, flash drive, or the like. Also, a person of skill in the art should recognize that the functionality of various computing devices may be combined or integrated into a single computing device, or the functionality of a particular computing device may be distributed across one or more other computing devices without departing from the scope of the exemplary embodiments of the present invention.

While has aspects of some embodiments of the present disclosure have been described in some detail with reference to some embodiments thereof, the disclosed embodiments described herein are not intended to be exhaustive or to limit the scope of the invention to the exact forms disclosed. Persons skilled in the art and technology to which this invention pertains will appreciate that alterations and changes in the described structures and methods of assembly and operation can be practiced without meaningfully departing from the principles, and scope of this invention, as set forth in the following claims.

## Claims

1. A method comprising:

    generating (110), by a processor (603), from a first conditional variational autoencoder (200) comprising a first latent space (207) and a first transformer decoder (202), a set of finger poses;
    generating (120), by the processor (603), from a second conditional variational autoencoder (200) comprising a second latent space (207) and a second transformer decoder (202), a set of wrist motions; and
    combining (130), by the processor (603), the set of finger poses and the set of wrist motions to generate a synthetic dataset of hand and arm gestures.

2. The method of claim 1, wherein the combining (130) comprises performing, by the processor (603), a Cartesian product of the set of finger poses and the set of wrist motions.

3. The method of claim 1 or 2, wherein the first conditional variational autoencoder (200) is different than the second conditional variational autoencoder (200).

4. The method of claim 3, wherein the first transformer decoder (202) has eight layers and the second transformer decoder (202) has two layers.

5. The method of any one of claims 1 to 4, further comprising generating, by the processor (603), a hand mesh model (403) of the hand and arm gestures.

6. The method of any one of claims 1 to 5, wherein the set of finger poses comprises at least one number gesture, at least one trigger gesture, and at least one special gesture.

7. A method comprising:

    generating (310), by a processor (603), a hand mesh model (403); and
    joining (320), by the processor (603), an arm mesh model (404) to the hand mesh model (403) to generate a hand-arm mesh model, wherein the joining the arm mesh model (404) to the hand mesh model (403) comprises:

identifying, by the processor (603), wrist boundary vertices (406, 407) of the hand mesh model (403) and the arm mesh model (404);

controlling, by the processor (603), a number of the wrist boundary vertices (406) of the hand mesh model (403) to be equal to a number of the wrist boundary vertices (407) of the arm mesh model (404); and

applying, by the processor (603), a wrist rotation matrix to the hand mesh model (403).

8. The method of claim 7, further comprising removing, by the processor (603), overlapping faces between the hand mesh model (403) and the arm mesh model (404) at a wrist of the hand-arm mesh model (404).

9. The method of claim 8, further comprising interpolating, by the processor (603), between the hand mesh model (403) and the arm mesh model (404) at the wrist to prevent visual seams between the hand mesh model (403) and the arm mesh model (404).

10. The method of claim 7 or 8, further comprising:

applying, by the processor (603), a skin texture to the hand mesh model (403); and

propagating (340), by the processor (603), the skin texture of the hand mesh model (403) to the arm mesh model (404).

11. The method of any one of claims 7 to 10, wherein the hand mesh model (403) comprises a NIMBLE model.

12. The method of any one of claims 7 to 11, wherein the arm mesh model (404) comprises a SMPL-X model.

13. The method of any one of claims 7 to 12, further comprising applying, by the processor (603), a global transformation to the hand-arm mesh model.

14. The method of any one of claims 7 to 13, wherein the generating the hand mesh model (403) comprises converting, by the processor (603), a MANO hand model to a NIMBLE hand model.

15. The method of any one of claims 7 to 14, wherein the hand mesh model (403) comprises a Handy model.

16. A method of simulating real-world camera configurations, the method comprising:

arranging a plurality of cameras in a hemispherical configuration around a hand-arm mesh model; and capturing hand motions of the hand-arm mesh model from different perspectives with the plurality of cameras.

17. The method of claim 16, wherein the plurality of cameras comprises a plurality of static cameras (501).

18. The method of claim 16 or 17, wherein the plurality of cameras comprises a plurality of dynamic cameras (502).

19. The method of claim 18, wherein the plurality of dynamic cameras (502) comprises a first camera having a close-up lens facing a palm side of the hand-arm mesh model, and a pair of stereo cameras facing a back side of the hand-arm mesh model.

20. The method of any one of claims 16 to 19, further comprising generating the hand-arm mesh model, comprising:

generating (310), by a processor (603), a hand mesh model (403); and

joining (320), by the processor (603), an arm mesh model (404) to the hand mesh model (403) to generate the hand-arm mesh model, wherein the joining the arm mesh model (404) to the hand mesh model (403) comprises:

identifying, by the processor (603), wrist boundary vertices (406) of the hand mesh model (403) and wrist boundary vertices (407) of the arm mesh model (404);

controlling, by the processor (603), a number of the wrist boundary vertices (406) of the hand mesh model (403) to be equal to a number of the wrist boundary vertices (407) of the arm mesh model (404); and

applying, by the processor (603), a wrist rotation matrix to the hand mesh model (403).

# FIG. 1

100

```
┌─────────┐
│  START  │
└─────────┘
```

110

GENERATING A SET OF FINGER POSE FROM A FIRST CONDITIONAL VARIATIONAL AUTOENCODER(CVAE)

120

GENERATING A SET OF WRIST MOTIONS FROM A SECOND CVAE

130

COMBINING THE FINGER POSES AND THE WRIST MOTIONS TO GENERATE A SYNTHETIC DATASET OF HAND AND ARM GESTURES

140

TRAINING A HAND POSE ESTIMATION MODEL AND/OR A HAND GESTURE RECOGNITION MODEL UTILIZING THE HAND AND ARM GESTURE DATABASE

```
┌─────────┐
│   END   │
└─────────┘
```

# FIG. 2

## FIG. 3

**LOCAL GESTURES**

**NUMBER**

| | |
|---|---|
| ONE/THUMB | ONE/INDEX |
| TWO | FIVE/MENU |
| CLOSED-TWO | SIX |
| THREE | SEVEN |
| FOUR | EIGHT |
| ZERO/FIRST | ZERO/CIRCLE |

**TRIGGER**

INDEX-TIP/PINCH
MIDDLE-TIP
RING-TIP
PINKY-TIP

**SPECIAL POSE**

| | | |
|---|---|---|
| SHAPE C | SNAP | CAPISCE |
| MESURE | GRAB | OK |
| FINGER-BEND | HEART | |
| FINGER-CROSS | PHONE-CALL | |

PALM/NEUTRAL

TRANSIT

**GLOBAL MOTIONS**

| MOVE | ROTATE |
|---|---|
| N/A | N/A |
| LEFT | LEFT |
| RIGHT | RIGHT |
| CIRCLE | DOWNWARD |
| V | UPWARD |
| CROSS | BACKWARD |
| DENY | FORWARD |
| WAVE | CLOCKWISE |
| KNOB | |
| GRAB | |
| PINCH | |

X

**LOCAL X GLOBAL**

=

ONE/INDEX

PALM-ROTATE-LEFT

PALM-TRANSIT-SHAPE C

INDEX-MOVE-CIRCLE

OK

CLOSED-TWO-ROTATE-LEFT

PALM-TRANSIT-ZERO/FIST

PALM-MOVE-CIRCLE

EP 4 641 355 A2

# FIG. 4

```
                                                    300

                    ( START )

                         |
                         v
              +---------------------------+  310
              | GENERATING A HAND MESH    |
              |         MODEL             |
              +---------------------------+
                         |
                         v
              +---------------------------+  320
              | ATTACHING A FOREARM MESH  |
              | MODEL TO THE HAND MESH    |
              |         MODEL             |
              +---------------------------+
                         |
                         v
              +---------------------------+  330
              | REMOVING OVERLAPPING      |
              | FACES BETWEEN THE HAND    |
              | MESH MODEL AND THE        |
              | FOREARM MESH MODEL        |
              +---------------------------+
                         |
                         v
              +---------------------------+  340
              | PROPAGATING A SKIN        |
              | TEXTURE OF THE HAND MESH  |
              | MODEL TO THE FOREARM      |
              | MESH MODEL                |
              +---------------------------+
                         |
                         v
                    ( END )
```

## FIG. 5A

SIMPL-X

SIMPL-X

400

401

402

400

## FIG. 5B

NIMBLE

403

UP-SAMPLE

404

# FIG. 5C

FIG. 5D

FIG. 5E

## FIG. 6A

EP 4 641 355 A2

FIG. 6B

502

502

502

502

404

403

F-FOLLOW

EGO

FIG. 6C

## FIG. 6D

FIG. 7A

# FIG. 7B

DIGITAL DISPLAY — 601

LENS SYSTEM — 602

POWER SUPPLY — 605

PROCESSOR — 603

COMMUNICATION MODULE — 607

NON-VOLATILE MEMORY DEVICE — 604

INPUT DEVICE — 606

600

EP 4 641 355 A2

FIG. 8

"TURN THE KNOB"

"..."

"..."

"DRAW A CHECK"

"DRAW A CIRCLE"

GESTURE VARIATION

BIOMECHANICAL PROJECTION

GESTURES AND MOTIONS

ARMS

APPEARANCES

ENVIRONMENT MAPS

"GESTURE: GRAB"

NEUTRAL

PEAK

700

702

701
703

704

707

705

705

706

EP 4 641 355 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63707422

**Non-patent literature cited in the description**

- **ROMERO et al.** Embodied Hands: Modeling and Capturing Hands and Bodies Together. *arXiv:2201.02610*, 07 January 2022
- **YANG et al.** CPF: Learning a Contact Potential Field to Model the Hand-Object Interaction. *International Conference on Computer Vision (ICCV)*, 2021
- **LI et al.** NIMBLE: A Non-rigid Hand Model with Bones and Muscles. *arXiv:2202.04533*, 18 July 2022
- **POTAMIAS et al.** Handy: Towards a high fidelity 3D hand shape and appearance model. *Proceedings of the IEEE/CVF Conference on Computer Vision and Pattern Recognition (CVPR)*, June 2023, 4670-4680
- **PAVLAKOS et al.** *Expressive Body Capture: 3D Hands, Face, and Body from a Single Image*